# EUROPÄISCHE PATENTSCHRIFT

(11) **EP 4 182 430 B1**
(45) Veröffentlichungstag und Bekanntmachung des Hinweises auf die Patenterteilung: **14.08.2024**
(21) Anmeldenummer: 21749543.1
(22) Anmeldetag: 14.07.2021
(51) Int. Cl.: C12M 1/04, C12M 1/00, C12M 1/12

(54) **VORRICHTUNG ZUR GEWÄHRLEISTUNG EINER STERILEN UMGEBUNG FÜR DIE INKUBATION VON ZELLKULTUREN**
APPARATUS FOR ENSURING A STERILE ENVIRONMENT FOR INCUBATING CELL CULTURES
DISPOSITIF POUR GARANTIR UN ENVIRONNEMENT STÉRILE POUR L'INCUBATION DE CULTURES DE CELLULES

(30) Priorität: 16.07.2020 DE 102020118898; 05.10.2020 DE 102020126038
(43) Veröffentlichungstag der Anmeldung: 24.05.2023
(73) Patentinhaber: S-Biosystems GmbH, 57072 Siegen (DE)
(72) Erfinder: THIEL, Erwin Richard, 57078 Siegen (DE); ZANDER, Christoph, 57258 Freudenberg (DE); IRLE, Stephan, 57074 Siegen (DE)
(74) Vertreter: Köllner & Partner mbB
(86) Internationale Anmeldenummer: PCT/EP2021/069642
(87) Internationale Veröffentlichungsnummer: WO 2022/013303

(56) Entgegenhaltungen:
- EP-A1- 1 403 363
- EP-A1- 2 873 724
- EP-A1- 3 778 853
- EP-A2- 0 808 657
- EP-A2- 1 552 888
- WO-A2-2011/130865
- DE-A1- 102006 004 157
- DE-A1- 102010 012 790
- DE-A1- 102014 106 877
- DE-B3- 102017 104 508
- US-A1- 2018 104 697
- US-A1- 2018 105 787

## Beschreibung

### Gebiet der Erfindung

Die Erfindung betrifft eine Vorrichtung zur Gewährleistung einer sterilen Umgebung für die Inkubation von Zellkulturen. Derartige Vorrichtungen dienen vorwiegend zum Kultivieren von Zellkulturen, überwiegend für medizinische und Forschungszwecke, wobei die Sterilität sämtlicher Elemente, die mit den Zellkulturen in Berührung kommen, und die Vermeidung jedweder Kontamination dieser Zellkulturen im Vordergrund stehen.

Durch die Erfindung der Gen-Schere (bekannt unter dem Stichwort CRISP/CAS) hat sich die Möglichkeit einer gezielten Veränderung von genetischem Material ergeben. Vor diesem Hintergrund sind bereits heute verschiedene Arbeitsgruppen dabei, neue zelltherapeutische Therapien zu entwickeln, die auf einer Manipulation von Gen-Material beruhen. Im Bereich humanmedizinischer Therapien wird es sich in diesem Zusammenhang um eine personalisierte Medizin handeln, bei der Patienten-Material (also lebende Zellen eines Patienten) vor und nach der gentechnischen Manipulation in Zellkulturen inkubiert werden. In der Absicht, Kontaminationen dieses Patienten-Materials strikt zu vermeiden, werden Vorrichtungen benötigt, die eine sterile Umgebung für diese Zellkulturen während der Phase der Inkubation sicher gewährleisten.

### Stand der Technik

Die Kultivierung von Zellkulturen erfolgt in Inkubatoren, in deren Inneren eine wählbare, konstante Temperatur (zumeist 37 °C), eine einstellbare CO₂-Konzentration (z.B. 5%), sowie eine hohe relative Luftfeuchtigkeit herrschen soll. Somit sind in einem Inkubator ideale Wachstumsbedingungen für jede Art von Zellen gegeben, also sowohl für erwünschte als auch für unerwünschte Kulturen.

Eine besonders kritische Komponente stellt in einem Inkubator die Wasserversorgung dar. Grundsätzlich hat das Wasser im Inkubator die Aufgabe, eine relativ hohe Luftfeuchtigkeit von nahe 100% zu gewährleisten, damit das wässrige Nährmedium, in dem sich die Zellen der Zellkultur befinden, nicht oder nur sehr wenig verdampft. Um ein Wachstum von zellbiologischen Verunreinigungen im Wasserreservoir zu verhindern, werden dem Wasser typischerweise für Einzeller toxische Substanzen beigemischt.

Aus dem zuvor beschriebenen ergeben sich gleich zwei Probleme: Zum einen kann mit dem Wasser eine Verunreinigung eingetragen werden, die sich dann im Inkubator vermehrt, auch wenn dem Wasser selbst eine toxische Substanz beigefügt wird. Zum anderen wird durch die Kombination der relativ hohen Luftfeuchtigkeit und der für ein Zellwachstum optimalen Temperatur jede ggf. vorhandene Kontamination zu einem explosionsartigen Wachstum angeregt, was sich bei einer Kondensation der Luftfeuchtigkeit im Inkubator noch um ein vielfaches verstärkt.

In der Praxis resultiert daher aus den idealen Wachstumsbedingungen im Inkubator, dass sich dort nicht nur die Zellen von Patienten vermehren, sondern ebenso auch unbeabsichtigt eingebrachte Fremdzellen, sogenannte Verunreinigungen oder Kontaminationen. Dies kann zu einer Verunreinigung bzw. Kontamination der eigentlichen Zellkultur, dem Patienten-Material, führen, was zur Folge hat, dass das Patienten-Material für eine Therapie unbrauchbar wird und im schlimmsten Fall der Patient an den Folgen der Kontamination verstirbt.

Mit dem Ziel, eine Kontamination des Inkubators durch Fremdzellen zu reduzieren, muss der Innenraum herkömmlicher Inkubatoren in regelmäßigen Abständen sterilisiert werden (Dekontamination). Zu diesem Zweck wird der Innenraum des Inkubators für eine Zeit von bis zu mehreren Stunden auf Temperaturen von nahe 200 °C gebracht. Auf diese Weise wird die Population dort vorhandener Zellen stark dezimiert, jedoch nicht völlig ausgelöscht. Eine Reduktion der Kontamination um z.B. einen Faktor Tausend ist üblich.

Ein anderer Ansatz, die Gefahren einer Kontamination zu verringern, besteht darin, dass sich die Proben in einem separaten, abgeschlossenen Behältnis befinden. Dieses Behältnis ist in der Regel mit einer gasdurchlässigen Membran versehen, so dass ein Gas- und Feuchtigkeits-Austausch mit der Atmosphäre im Inkubator gegeben ist. Die Membran selbst ist in der Regel als Sterilfilter ausgelegt, wodurch ein Eindringen von biologischen Kontaminationen ins Innere des Behältnisses weitgehend minimiert wird. Allerdings lassen sich mit diesen Behältnissen nicht die Kontaminationen im Inkubator selbst und vor allem nicht auf der Außenfläche eines derartigen Behältnisses verhindern. Insbesondere auf der Außenfläche des Sterilfilters eines solchen Behältnisses können sich bevorzugt Kontaminationen ablagern, so dass ein Verschleppen dieser Kontamination in den Innenraum des Behältnisses nicht auszuschließen ist.

Um Parameter wie Temperatur oder CO₂-Konzentration im Inkubator zu überwachen bzw. zu regulieren, ist es notwendig, Sensoren in dessen Innenraum zu platzieren. Viele dieser Sensoren reagieren jedoch empfindlich gegenüber hohen Temperaturen, beispielsweise vertragen die üblichen CO₂-Sensoren keine Temperaturen von über 160 °C. Aus diesem Grund ist eine Dekontamination eines Inkubators mit Aufwand verbunden, da zuvor zumindest einige der Sensoren aus dem Inkubator entfernt werden müssen. Außerdem können diese Sensoren zwar nicht selbst dekontaminiert werden, müssen aber dennoch wieder in den Innenraum des Inkubators eingebaut werden, wodurch die Wirkung der Dekontamination desselben zumindest teilweise wieder zunichte gemacht wird.

Für viele derzeitige Anwendungen in der Zellbiologie und der Medizin und besonders für mögliche zukünftige Anwendungen, beispielsweise in der Therapie von genetisch bedingten Krankheiten (z.B. Tumoren, Leukämie oder Erbkrankheiten), ist eine vollständig sterile Umgebung im Inkubator unabdingbar.

Aus der Veröffentlichung WO 2015/172882 A1 ist ein System bekannt, das durch voneinander autarke Mini-Inkubatoren, die jeweils für nur einen Probenträger ausgelegt sind, die Sicherheit im Labor erhöhen und insbesondere Konfusion und mögliche Kontamination der Proben verhindern soll. Vollständige Sterilität kann dieses System jedoch nicht garantieren.

Die Veröffentlichung WO 2011/130865 A2 beschreibt einen im Wesentlichen herkömmlich ausgelegten Inkubator, bei dem zum Verhindern von Kontamination der Proben vorgesehen ist, möglichst viele Behandlungsschritte innerhalb des Inkubators maschinell, ohne menschliche Beteiligung auszuführen. Hierzu sind spezielle Vorrichtungen wie z.B. Roboterarme vorgesehen. Vollständige Sterilität kann jedoch auch hierbei nicht gewährleistet werden.

DE 10 2006 004157 offenbart ein Verfahren sowie einen Inkubator mit einer oder mehreren Kultivierungskammern zum Inkubieren von Zellen. Ein abgeschlossenes Kulturgefäß, wie beispielsweise eine Kulturflasche, kann im Wärmeraum des Inkubators eingebracht und integriert werden. Somit schließt der Behälter die spezifische Zellkultur steril gegen die Umgebung ab und ermöglicht somit eine gegen unerwünschte Störung abgeschirmte, individuelle Zellkultur.

### Aufgabe

Aufgabe der Erfindung ist es, eine Vorrichtung anzugeben, die eine Verbesserung der Sterilität bei der Inkubation von Zellkulturen ermöglicht.

### Lösung

Diese Aufgabe wird durch die Gegenstände der unabhängigen Ansprüche gelöst. Vorteilhafte Weiterbildungen der Gegenstände der unabhängigen Ansprüche sind in den Unteransprüchen gekennzeichnet. Der Wortlaut sämtlicher Ansprüche wird hiermit durch Bezugnahme zum Inhalt dieser Beschreibung gemacht.

Die Verwendung der Einzahl soll die Mehrzahl nicht ausschließen, was auch im umgekehrten Sinn zu gelten hat, soweit nichts Gegenteiliges offenbart ist.

Zur Lösung der Aufgabe wird eine Vorrichtung zur Gewährleistung einer sterilen Umgebung für die Inkubation von Zellkulturen vorgeschlagen. Die Vorrichtung hat einen abgedichteten Wärmeraum, der mindestens ein Heizelement aufweist. Der Wärmeraum weist Sensoren für Temperatur und/oder Luftfeuchtigkeit und/oder CO₂ und au-ßen mindestens einen Anschluss für eine Gaszufuhr auf. Der Anschluss ist mit einer Leitung verbunden, die im Innenraum des Wärmeraums einen Gasanschluss bereitstellt. Bei der Gaszufuhr handelt es sich typischerweise um CO₂, für spezielle Zellkulturen können aber auch andere Gase vorgesehen sein. Die Vorrichtung hat außerdem einen abgedichteten Kultivierungsbehälter zum Aufnehmen der Zellkulturen, der im Wärmeraum angeordnet ist. Der Kultivierungsbehälter hat keine eigene Heizung. Der Kultivierungsbehälter weist für jeden der genannten Sensoren einen abdichtbaren Durchlass auf, der in Kommunikation mit der Atmosphäre im Kultivierungsbehälter steht. Der Kultivierungsbehälter weist auch mindestens einen abdichtbaren Gas-Ein- und/oder Auslass auf, der mit dem mindestens einen Gasanschluss im Innenraum des Wärmeraums koppelbar ist. Die Sensoren des Wärmeraums sind so angeordnet, dass sie beim Einsetzen des Kultivierungsbehälters in den Wärmeraum in die jeweiligen abdichtbaren Durchlässe des Kultivierungsbehälters eingebracht werden. Auf diese Weise können die Sensoren des Wärmeraums Messdaten zu den Bedingungen im Kultivierungsbehälter liefern. Die Vorrichtung hat des Weiteren ein Wasserreservoir, welches im Kultivierungsbehälter angeordnet ist.

Mit dieser Vorrichtung lässt sich eine erhebliche Verbesserung der Sterilisierungssituation bei der Inkubation von Zellkulturen erreichen, da die drei Elemente Wärmeraum, Kultivierungsbehälter und Wasserreservoir getrennt voneinander mit dem dafür jeweils optimalen Verfahren sterilisiert werden können. Dadurch, dass die Sensoren sich nicht im Kultivierungsbehälter befinden, kann dieser z.B. bei Bedarf durch hohe Temperaturen (z.B. über 200 °C) dekontaminiert werden. Durch den - seinerseits sterilisierten - Wärmeraum ist besonders sichergestellt, dass die Zellkulturen im Kultivierungsbehälter weder von außen kontaminiert werden können, noch ihrerseits Kontaminationen im Labor verursachen können.

Durch den abdichtbaren Gas-Ein- und/oder Auslass, der an den im Inneren des Wärmeraums bereitgestellten Gasanschluss gekoppelt werden kann, ist die Aufrechterhaltung optimaler Bedingungen im Kultivierungsbehälter möglich. Diese Bedingungen können mittels Sensoren, die in den bzw. die abdichtbaren Durchlässe passen, kontrolliert werden.

Eine mögliche Kontamination der Zellkulturen im Kultivierungsbehälter durch die Sensoren kann dadurch verhindert werden, dass jeder der genannten Sensoren des Wärmeraums durch einen Sterilfilter abgedeckt ist.

Ebenso können Kontaminierungen durch die Gaszufuhr dadurch vermieden werden, dass der mindestens eine Anschluss des Wärmeraums für eine Gaszufuhr, die damit verbundene Leitung oder der über diese Leitung verbundene, im Innenraum des Wärmeraums bereitgestellte Gasanschluss einen Sterilfilter aufweist.

Zum Einbringen des Kultivierungsbehälters in den Wärmeraum ist es vorteilhaft, wenn der Wärmeraum einen dicht schließenden Deckel oder eine dicht schließende Tür aufweist, wobei die Öffnung des Deckels oder der Tür einen Querschnitt hat, der größer ist als eine Breite des Kultivierungsbehälters.

Kontaminierungen durch die Sensoren können auch dadurch verhindert werden, dass jeder abdichtbare Durchlass am Kultivierungsbehälter einen Sterilfilter aufweist. Diese Ausführung wird besonders bevorzugt. Sie kann alternativ zu Sterilfiltern direkt an den Sensoren des Wärmeraums oder ergänzend dazu eingesetzt werden.

Kontaminierungen durch die Gaszufuhr können bevorzugt dadurch verhindert werden, dass der mindestens eine abdichtbare Gas-Ein- und/oder Auslass am Kultivierungsbehälter einen Sterilfilter aufweist. Dieser kann wiederum entweder alternativ zu Sterilfiltern direkt am Gas-Anschluss des Wärmeraums oder ergänzend dazu eingesetzt werden.

Um sicherzustellen, dass der Kultivierungsbehälter vor der Benutzung sterilisiert wurde, ist es von Vorteil, wenn der Kultivierungsbehälter ein Einweg-Element ist. Dieses kann bereits unter sterilen bzw. Reinraum-Bedingungen hergestellt werden.

Das Einbringen des bzw. der Probenträger mit den Zellkulturen wird erleichtert, wenn der Kultivierungsbehälter einen dicht schließenden Deckel oder eine dicht schließende Tür aufweist.

Besonders vorteilhaft ist es, wenn das Wasserreservoir mit einer semipermeablen Membran bedeckt ist. Diese Membran sollte wasserundurchlässig sein, aber Wasserdampf durchlassen. Vliesstoffe aus Polyethylen hoher Dichte (PE-HD) haben sich beispielsweise für diesen Zweck hervorragend bewährt. Auf diese Weise kann das Wasser bereits bei der Herstellung des Wasserreservoirs hineingegeben werden.

Ein vorzeitiges Verdunsten des Wassers aus dem Wasserreservoir kann verhindert werden, wenn die semipermeable Membran des Wasserreservoirs vor der Nutzung mit einer für Wasser und Wasserdampf undurchlässigen Folie, z.B. mit einer Kunststofffolie, versiegelt ist, die zur Benutzung entfernt wird.

Eine mögliche Kontamination der Zellkulturen über das Wasserreservoir wird besonders unwahrscheinlich, wenn das Wasserreservoir steriles Reinstwasser enthält.

Die Sterilität des Wasserreservoirs kann besonders gut sichergestellt werden, wenn das Wasserreservoir ein Einweg-Element ist.

Die Inkubationsbedingungen im Kultivierungsbehälter können besonders optimal eingestellt werden, wenn eine Steuerung und/oder Regelung vorhanden ist, welche in der Lage ist, die Bedingungen im Kultivierungsbehälter anhand von Daten der Sensoren des Wärmeraums zu beeinflussen. Dabei dienen die Daten des Temperatursensors zur Steuerung und/oder Regelung der Heizelemente des Wärmeraums und die Daten des CO₂-Sensors zur Steuerung und/oder Regelung der mindestens einen Gaszufuhr. Die Daten des Luftfeuchtigkeits-Sensors dienen zur Ausgabe von Warnmeldungen, welche den Nutzer z.B. auffordern können, das Wasserreservoir zu erneuern, wenn die Luftfeuchtigkeit einen zu niedrigen Wert erreicht.

Die Aufgabe wird ferner gelöst durch einen abgedichteten Kultivierungsbehälter zum Aufnehmen von Zellkulturen. Der Kultivierungsbehälter weist mindestens einen abdichtbaren Durchlass für einen Sensor auf, der in Kommunikation mit der Atmosphäre im Kultivierungsbehälter steht. Ferner weist der Kultivierungsbehälter mindestens einen abdichtbaren Gas-Ein- und/oder Auslass auf, der mit einem Gasanschluss koppelbar ist. Schließlich befindet sich im Inneren des Kultivierungsbehälters eine Position für ein Wasserreservoir.

Ein solcher Kultivierungsbehälter kann problemlos dekontaminiert werden, und/oder sogar unter sterilen Bedingungen hergestellt werden. Kontaminationssensitive Proben können somit in einen solchen Kultivierungsbehälter eingesetzt werden und sind dann für den gesamten Kultivierungsvorgang besonders geschützt. Der Kultivierungsbehälter kann dann in einen Wärmeraum, wie er bereits beschrieben wurde, eingesetzt werden. Durch den abdichtbaren Gas-Ein- und/oder Auslass, der an den im Inneren des Wärmeraums bereitgestellten Gasanschluss gekoppelt werden kann, ist die Aufrechterhaltung optimaler Bedingungen im Kultivierungsbehälter möglich. Diese können mittels Sensoren, die in den bzw. die abdichtbaren Durchlässe passen, kontrolliert werden.

Dieser Kultivierungsbehälter kann somit in einer Vorrichtung, wie sie oben bereits beschrieben wurde, verwendet werden.

Weitere Einzelheiten und Merkmale ergeben sich aus der nachfolgenden Beschreibung von bevorzugten Ausführungsbeispielen in Verbindung mit den Figuren. Hierbei können die jeweiligen Merkmale für sich alleine oder zu mehreren in Kombination miteinander verwirklicht sein. Die Möglichkeiten, die Aufgabe zu lösen, sind nicht auf die Ausführungsbeispiele beschränkt. So umfassen beispielsweise Bereichsangaben stets alle - nicht genannten - Zwischenwerte und alle denkbaren Teilintervalle.

Ein Ausführungsbeispiel ist in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente. Im Einzelnen zeigt:
- Fig. 1: eine schematische Gesamtdarstellung einer erfindungsgemäßen Vorrichtung;
- Fig. 2: eine schematische Darstellung eine Wärmeraums einer erfindungsgemäßen Vorrichtung;
- Fig. 3: eine schematische Darstellung eines Kultivierungsbehälters einer erfindungsgemäßen Vorrichtung; und
- Fig. 4: eine schematische Darstellung eines Wasserreservoirs einer erfindungsgemäßen Vorrichtung.

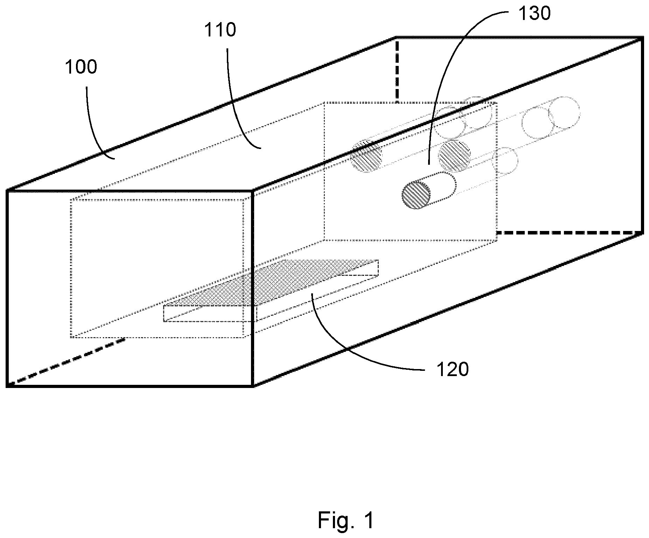

Fig. 1 zeigt eine Vorrichtung zur Gewährleistung einer sterilen Umgebung für die Inkubation von Zellkulturen in Gesamtdarstellung, so dass die Anordnung der drei Hauptkomponenten zueinander ersichtlich wird. Der Wärmeraum 100 ist das äußere Element und enthält im normalen Betrieb den Kultivierungsbehälter 110. Dieser kann, je nachdem, was für Kulturen inkubiert werden sollen, unterschiedlich groß ausgeführt sein. Bevorzugt wird eine Ausführung, bei der der Kultivierungsbehälter einen Probenträger, z.B. eine Mikrotiterplatte oder eine Petrischale, aufnehmen kann. Es sind aber selbstverständlich auch größere Ausführungen möglich.

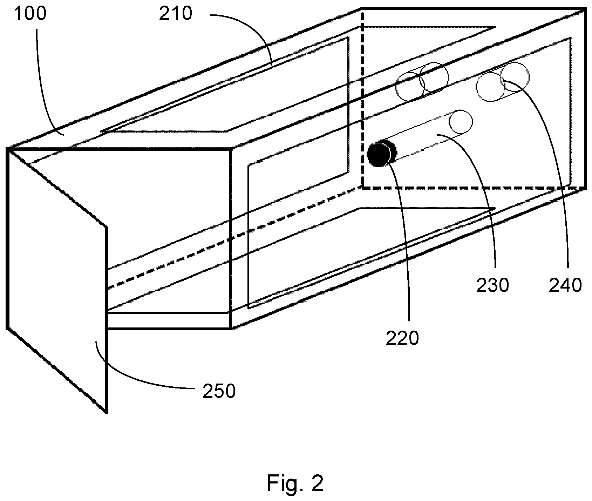

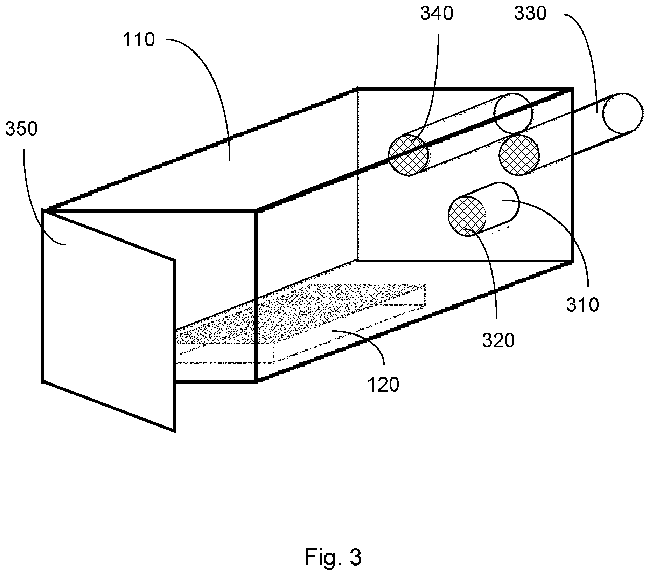

Im Inneren des Kultivierungsbehälters ist das Wasserreservoir 120 angeordnet. In der gewählten Darstellung hinter dem Kultivierungsbehälter sind Anschlüsse bzw. Verbindungselemente 130 angeordnet, die den Kultivierungsbehälter 110 an den Wärmeraum 100 anschließen. Diese Anschlüsse bzw. Verbindungselemente werden im Folgenden im Zusammenhang mit Fig. 2 und 3 detaillierter beschrieben.

In Fig. 2 ist der Wärmeraum 100 dargestellt. Dieser bildet eine äußere Hülle, die die gewünschte Temperatur gewährleistet. Zu diesem Zweck enthält der Wärmeraum Heizelemente 210. Im Wärmeraum 100 befinden sich auch die erforderlichen Sensoren, z.B. für die Temperatur, relative Luftfeuchte oder den CO₂-Gehalt.

In Fig. 2 ist wegen der Übersichtlichkeit nur der CO₂-Sensor 220 dargestellt. Dieser ist an einer Halterung 230 angebracht, die den Sensor so positioniert, dass er beim Einsetzen des Kultivierungsbehälters an die richtige Stelle gelangt. Ferner ist mindestens ein Anschluss für eine Gaszufuhr vorhanden. Dieser Anschluss ist mit einer Leitung verbunden, die im Inneren des Wärmeraums einen Gasanschluss 240 bereitstellt. Bevorzugt sind zwei derartige Gasanschlüsse vorhanden, von denen beispielsweise einer eine CO₂-Zufuhr ermöglicht, und der andere eine Möglichkeit zur Gasentnahme bietet.

Vorzugsweise gegenüber von den Sensoren und Gasanschlüssen ist eine Tür 250 vorhanden, die gasdicht schließend ausgeführt ist. Alternativ dazu kann beispielsweise auch ein abnehmbarer Deckel o.ä. vorgesehen sein. Die Öffnung des Deckels oder der Tür 250 sollte einen Querschnitt haben, der groß genug ist, dass der Kultivierungsbehälter 110 problemlos eingesetzt werden kann.

Um eine Kontamination der Laborumgebung durch das Einbringen des Wärmeraums 100 in das Labor zu vermeiden, ist es von Vorteil, wenn der Wärmeraum vor dem Verbringen in das Labor gereinigt und in eine Verpackung gegeben wird, in der dieser sterilisiert werden kann. Die Verpackung besteht zumindest teilweise aus einer semipermeablen Membran, die gasdurchlässig, aber hinreichend feinporig ist, so dass keine Keime hindurchkönnen. Das Sterilisieren kann dann z.B. in bekannter Weise durch Begasen mit Ethylenoxid erfolgen.

Fig. 3 zeigt den Kultivierungsbehälter 110. Dieser wird in den Wärmeraum eingesetzt und kann daraus wieder entnommen werden. Der Kultivierungsbehälter weist für die Sensoren mindestens einen abdichtbaren Durchlass 310 auf, der in Kommunikation mit der Atmosphäre im Kultivierungsbehälter steht. Dieser Durchlass 310 ist vorzugsweise an seiner Innenseite durch einen Sterilfilter 320 abgedeckt, so dass der Kultivierungsbehälter gegen Kontaminationen von außen geschützt ist. Die Sensoren des Wärmeraums sind so angeordnet, dass sie beim Einsetzen des Kultivierungsbehälters 110 in den Wärmeraum in die jeweiligen abdichtbaren Durchlässe des Kultivierungsbehälters eingebracht werden. Dadurch können sie Daten über die Zustände im Inneren des Kultivierungsbehälters 110 liefern. Der Kultivierungsbehälter weist auch mindestens einen abdichtbaren Gas-Ein- und/oder Auslass 330 auf, der an den mindestens einen Gasanschluss im Innenraum des Wärmeraums angepasst ist. Jeder dieser Gas-Ein- und/oder Auslässe besitzt vorzugsweise einen Sterilfilter 340, so dass der Kultivierungsbehälter 110 gegen Kontaminationen von außen geschützt ist, aber auch, damit eine Kontamination aus dem Kultivierungsbehälter in die Umgebung nicht erfolgen kann. Dies kann z.B. erforderlich sein, wenn sich infektiöses Material in der Zellkultur befindet. Vorzugsweise sind jeweils ein Gas-Einlass und ein Gas-Auslass vorhanden, wodurch sich ein Gasaustausch im Inneren des Kultivierungsbehälters leichter verwirklichen lässt.

Des Weiteren besitzt der Kultivierungsbehälter 110 eine Öffnung, durch die beispielsweise Probenträger mit Zellkulturen in den Kultivierungsbehälter eingebracht oder daraus entnommen werden können. Diese Öffnung kann mittels eines Deckels oder einer Tür 350 verschlossen werden. Der Verschluss durch den Deckel oder die Tür 350 sollte so ausgeführt sein, dass der Kultivierungsbehälter 110 gasdicht gegen die Umgebung abgeschlossen wird. Im Inneren des Kultivierungsbehälters ist ferner vorgesehen, ein Wasserreservoir 120 unterzubringen.

Um das Ziel zu erreichen, eine sterile Umgebung für die Inkubation von Zellkulturen zu gewährleisten, ist vorgesehen, dass der Kultivierungsbehälter 110 vor seiner Benutzung gereinigt und sterilisiert wird. Hierbei ist es von Vorteil, wenn es sich bei dem Kultivierungsbehälter 110 um einen Einwegartikel handelt. Die für Kontaminationen besonders kritischen Elemente, nämlich die Durchlässe 310 für die Sensoren, die Gas-Ein- bzw. Auslässe 330 sowie die zugehörigen Sterilfilter 320, 340 sind Teil des Kultivierungsbehälters. Sie werden also mitgeliefert, mit sterilisiert und auch mit entsorgt.

Auch für den Kultivierungsbehälter 110 bietet es sich an, eine spezielle Verpackung zum Sterilisieren vorzusehen, z.B. mit einer semipermeablen Membran. Als Sterilisierungsverfahren können unterschiedliche Verfahren angewendet werden, z.B. Gamma-Bestrahlung oder Begasung mit Ethylenoxid, oder auch thermische Verfahren.

Für eine Verwendung des Kultivierungsbehälters 110 in einer Reinraumumgebung ist es zudem von Vorteil, wenn die gesamte Herstellung des Kultivierungsbehälters, einschließlich der sterilen Verpackung, selbst in einem Reinraum erfolgt. Die fertige Komponente (also Kultivierungsbehälter 110 in der Sterilverpackung) kann dann noch mit einer reinraumtauglichen Umverpackung (z.B. Kunststoffbeutel) versehen werden.

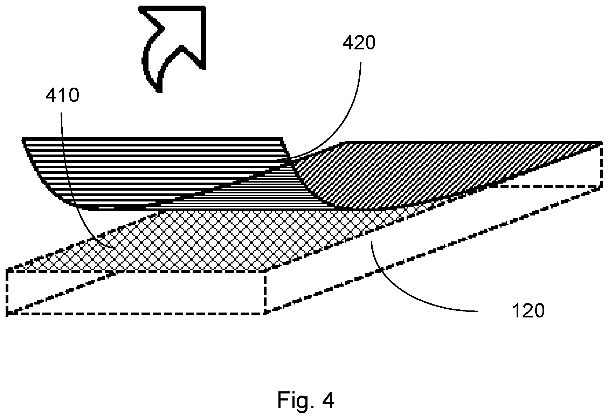

In Fig. 4 ist als weitere Komponente der erfindungsgemäßen Vorrichtung ein Wasserreservoir 120 schematisch dargestellt. Dieses muss in der Realität nicht unbedingt quaderförmig ausgeführt sein. Eine zylindrische oder blasenförmige Ausgestaltung sind beispielsweise ebenfalls vorstellbar. Das Wasserreservoir 120 weist vorzugsweise an mindestens einem Teil seiner Oberfläche eine semipermeable Membran 410 auf, z.B. eine Tyvek-Folie oder ähnliches. Die semipermeable Membran ermöglicht es, dass gasförmiger Wasserdampf aus dem Wasserreservoir in die Atmosphäre im Kultivierungsbehälter entweicht, das flüssige Wasser aus dem Wasserreservoir jedoch nicht auslaufen kann.

Bevor das Wasserreservoir in den Kultivierungsbehälter eingesetzt wird, ist die semipermeable Membran vorzugsweise durch eine Folie 420, z.B. eine wasser- und gasdichte Kunststofffolie, versiegelt. Durch den Pfeil in Fig. 4 wird das Entfernen dieser versiegelnden Folie 420 vor dem Einsetzen des Wasserreservoirs angedeutet.

Besonders vorteilhaft ist es, wenn das Wasserreservoir 120 so ausgelegt ist, dass es separat, als Einweg-Reservoir, in den Kultivierungsbehälter 110 eingebracht werden kann. Das Wasserreservoir 120 kann in diesem Fall bereits bei der Herstellung mit Wasser befüllt werden. Bevorzugt wird dabei steriles Reinstwasser verwendet.

Um das Ziel zu erreichen, eine sterile Umgebung für die Inkubation zu gewährleisten, ist es von Vorteil, wenn das Wasserreservoir 120 vor seiner Benutzung von außen sterilisiert wird. Das Wasserreservoir kann zu diesem Zweck - wie auch die anderen Komponenten Wärmeraum 100 und Kultivierungsbehälter 110 - in einer Verpackung zum Sterilisieren untergebracht werden, z.B. in einer Verpackung mit einer semipermeablen Membran. Als Sterilisationsverfahren können dabei unterschiedliche Verfahren angewendet werden, z.B. Gamma-Bestrahlung oder Begasung mit Ethylenoxid.

Für eine Verwendung des Wasserreservoirs 120 in einer Reinraumumgebung ist es zudem von Vorteil, wenn die gesamte Herstellung des Wasserreservoirs, inklusive des Wassers und der sterilen Verpackung, in einem Reinraum erfolgt und die gesamte Komponente (also Wasserreservoir 120, Wasser, Wasserdampfdurchlässige Membran 410, Versiegelung 420 und Sterilverpackung) noch einmal in einem reinraumtauglichen Behältnis (z.B. KunststoffBeutel) verpackt wird.

Die oben beschriebene Vorrichtung bietet folgende Vorteile gegenüber dem Stand der Technik:
Dadurch, dass der Gaseinlass des Kultivierungsbehälters derart an den Wärmeraum angepasst ist, dass ein Gasaustausch zwischen Wärmeraum und Kultivierungsbehälter nicht stattfindet, ist eine Kontamination des Sterilfilters aus dem Bereich des Wärmeraums nicht möglich. Der Gas-Ein- bzw. Auslass lässt lediglich Gas vom Gasanschluss zum Kultivierungsbehälter durch.

In gleicher Weise ist der Gasauslass des Kultivierungsbehälters derart an den Wärmeraum angepasst, so dass ein Gasaustausch zwischen Kultivierungsbehälter und Wärmeraum unterbunden ist. Auf diese Weise ist eine Kontamination des Wärmeraums aus dem Bereich des Kultivierungsbehälters nicht möglich.

Des Weiteren bietet die erfindungsgemäße Vorrichtung den Vorteil, dass sich die Sensoren außerhalb des Kultivierungsbehälters befinden und somit nicht von den Proben im Kultivierungsbehälter kontaminiert werden können, da sie hiervon durch einen Sterilfilter getrennt sind.

Ferner ist durch die Verwendung eines sterilisierten Kultivierungsbehälters eine Kontamination von Proben ausgeschlossen. Dies gilt insbesondere, wenn es sich bei dem Kultivierungsbehälter um einen sterilisierten Einmal-Artikel handelt.

Durch die Verwendung von Reinstwasser, dass in einem sterilisierten Einweg-Reservoir in den sterilisierten Kultivierungsbehälter eingebracht wird, ist auch eine Kontamination durch das eingeführte Wasser ausgeschlossen. Diese Gefahr besteht auch nicht für den Fall, dass die relative Luftfeuchtigkeit so hoch ist, dass Wasser im Kultivierungsbehälter kondensiert, da alle Komponenten vor ihrer Benutzung sterilisiert wurden.

### Glossar

### Inkubator

Ein Inkubator, oder auch Brutschrank, ist ein Gerät, mit dem in der Biologie kontrollierte Außenbedingungen für verschiedene Entwicklungs- und Wachstumsprozesse geschaffen und erhalten werden können. Er dient der Schaffung und Erhaltung eines Mikroklimas mit eng geregelten Luftfeuchtigkeits- und Temperatur-Bedingungen. Ein Inkubator verfügt über eine Zeitschaltuhr und einen Temperaturregler und unter Umständen eine Einstellung für die Regelung der zugeführten Frischluft. Die eingestellte Temperatur ist dabei auf das Temperaturoptimum der zu inkubierenden Mikroorganismen abgestimmt. Zur Kultivierung tierischer Zellen werden CO₂-Inkubatoren verwendet. (Nach de.wikipedia.org/wiki/Inkubator_(Biologie))

### Mikrotiterplatte / Microplate

Eine Mikrotiterplatte (engl. *microwell plate,* von engl. *well* für Schacht, oder auch *microplate*) ist ein multipler Probenträger. Die i.d.R. rechteckigen Mikrotiterplatten bestehen meist aus Kunststoff, für sehr spezielle Anwendungen auch aus Glas. Sie enthalten zwischen 6 (2×3) und 1536 (32×48) voneinander isolierte Näpfchen (Kavitäten, engl. *wells*) in Reihen und Spalten. Die genauen Abmessungen (Länge×Breite×Höhe) betragen gemäß ANSI-Standard auf Empfehlung der Society for Biomolecular Screening (SBS) 127,76 × 85,48 ×14,35 mm. Mikrotiterplatten werden für die unterschiedlichsten mikrobiologischen Arbeitsgänge eingesetzt. Typische Einsatzbereiche sind die Zellzüchtung oder das Screening technischer Bioreaktionen. Durch die große Anzahl der Kavitäten und die Verwendung gleicher Typen eignen sich Mikrotiterplatten für die parallele Kultivierung und Testung einer Vielzahl unterschiedlicher Proben. Bedingt durch die normierte Größe können fast alle Arbeitsgänge mit geeigneten Robotern automatisiert werden. (Nach de.wikipedia.org/wiki/Mikrotiterplatte)

### Probenträger

Mikrobiologische Proben bzw. Zellkulturen werden in Behältern aufbewahrt, die hier als Probenträger bezeichnet werden. Je nach Art der Proben kann es sich dabei um die verschiedensten Behälter handeln. Typischerweise finden jedoch Petrischalen oder Mikrotiterplatten (sog. Microplates), teilweise auch Erlenmeyerkolben o.ä., Verwendung. Multiple Probenträger, beispielsweise Mikrotiterplatten, werden auch als Probenträgersysteme bezeichnet.

### Sterilfilter

Bei der Sterilfiltration werden Mikroorganismen aus dem Sterilisiergut durch Filtration abgeschieden. Als Filter werden meistens Membranen mit einem Porendurchmesser von 0,1 bis 0,22 µm verwendet. Sterilfiltration wird oftmals zur Sterilisierung hitzeempfindlicher Lösungen, beispielsweise serumhaltiger Gewebekulturlösungen, eingesetzt. Hauptanwendungen sind die Sterilfiltration von wässrigen Lösungen, hitzeempfindlichen Nährlösungen, Vitaminlösungen, Seren, Virusimpfstoffen, Plasmafraktionen und Proteinlösungen. (Nach https://de.wikipedia.org/wiki/Sterilisation#Sterilfiltration)

### Bezugszeichen

- 100: Wärmeraum
- 110: Kultivierungsbehälter
- 120: Wasserreservoir
- 130: Anschlüsse bzw. Verbindungselemente

- 210: Heizelement
- 220: CO₂-Sensor
- 230: Halterung für Sensor
- 240: Gasanschluss
- 250: Tür des Wärmeraums

- 310: Durchlass für Sensor
- 320: Sterilfilter für Sensor
- 330: Gas-Ein- bzw. Auslass
- 340: Sterilfilter für Gas-Ein- bzw. Auslass
- 350: Tür des Kultivierungsbehälters

- 410: semipermeable Membran
- 420: Folie

### zitierte Literatur

### zitierte Patentliteratur

WO 2015/172882 A1
WO 2011/130865 A2

## Patentansprüche

1. Vorrichtung zur Gewährleistung einer sterilen Umgebung für die Inkubation von Zellkulturen mit:
einem abgedichteten Wärmeraum (100):
wobei der Wärmeraum (100) mindestens ein Heizelement (210) aufweist;
wobei der Wärmeraum (100) Sensoren für Temperatur und/oder Luftfeuchtigkeit und/oder CO₂ (220) aufweist;
wobei der Wärmeraum (100) außen mindestens einen Anschluss für eine Gaszufuhr aufweist, wobei der Anschluss mit einer Leitung verbunden ist, die im Innenraum des Wärmeraums einen Gasanschluss (130; 240) bereitstellt;
einem abgedichteten Kultivierungsbehälter (110) zum Aufnehmen der Zellkulturen:
wobei der Kultivierungsbehälter (110) im Wärmeraum (100) angeordnet ist;
wobei der Kultivierungsbehälter (110) für jeden der genannten Sensoren des Wärmeraums einen abdichtbaren Durchlass (130; 310) aufweist, der in Kommunikation mit der Atmosphäre im Kultivierungsbehälter steht;
wobei der Kultivierungsbehälter (110) mindestens einen abdichtbaren Gas-Ein- und/oder Auslass (130; 330) aufweist, der mit dem mindestens einen Gasanschluss (130; 240) im Innenraum des Wärmeraums koppelbar ist;
wobei die Sensoren des Wärmeraums (100) derart angeordnet sind, dass sie beim Einsetzen des Kultivierungsbehälters (110) in den Wärmeraum (100) in die jeweiligen abdichtbaren Durchlässe (130; 310) des Kultivierungsbehälters eingebracht werden; und einem Wasserreservoir (120):
wobei das Wasserreservoir (120) im Kultivierungsbehälter (110) angeordnet ist.

2. Vorrichtung nach dem vorhergehenden Anspruch,
**dadurch gekennzeichnet,**
**dass** jeder der genannten Sensoren des Wärmeraums (100) durch einen Sterilfilter abgedeckt ist.

3. Vorrichtung nach einem der vorhergehenden Ansprüche,
**dadurch gekennzeichnet,**
**dass** der mindestens eine Anschluss (240) des Wärmeraums für eine Gaszufuhr, die damit verbundene Leitung oder der über diese Leitung verbundene, im Innenraum des Wärmeraums bereitgestellte Gasanschluss (130) einen Sterilfilter aufweist.

4. Vorrichtung nach einem der vorhergehenden Ansprüche,
**dadurch gekennzeichnet,**
**dass** der Wärmeraum (100) einen dicht schließenden Deckel oder eine dicht schließende Tür (250) aufweist;
wobei die Öffnung des Deckels oder der Tür (250) einen Querschnitt hat, der größer ist als eine Breite des Kultivierungsbehälters (110).

5. Vorrichtung nach einem der vorhergehenden Ansprüche,
**dadurch gekennzeichnet,**
**dass** jeder abdichtbare Durchlass (130; 310) am Kultivierungsbehälter (110) einen Sterilfilter (320) aufweist.

6. Vorrichtung nach einem der vorhergehenden Ansprüche,
**dadurch gekennzeichnet,**
**dass** der mindestens eine abdichtbare Gas-Ein- und/oder Auslass (130; 330) am Kultivierungsbehälter (110) einen Sterilfilter (340) aufweist.

7. Vorrichtung nach einem der vorhergehenden Ansprüche,
**dadurch gekennzeichnet,**
**dass** der Kultivierungsbehälter (110) ein Einweg-Element ist.

8. Vorrichtung nach einem der vorhergehenden Ansprüche,
**dadurch gekennzeichnet,**
**dass** der Kultivierungsbehälter (110) einen dicht schließenden Deckel oder eine dicht schließende Tür (350) aufweist.

9. Vorrichtung nach einem der vorhergehenden Ansprüche,
**dadurch gekennzeichnet,**
**dass** das Wasserreservoir (120) mit einer semipermeablen Membran (410) bedeckt ist.

10. Vorrichtung nach dem unmittelbar vorhergehenden Anspruch,
**dadurch gekennzeichnet,**
**dass** die semipermeable Membran (410) des Wasserreservoirs (120) vor der Nutzung mit einer für Wasser und Wasserdampf undurchlässigen Folie (420) versiegelt ist, die zur Benutzung entfernt wird.

11. Vorrichtung nach einem der vorhergehenden Ansprüche,
**dadurch gekennzeichnet,**
**dass** das Wasserreservoir (120) steriles Reinstwasser enthält.

12. Vorrichtung nach einem der vorhergehenden Ansprüche,
**dadurch gekennzeichnet,**
**dass** das Wasserreservoir (120) ein Einweg-Element ist.

13. Vorrichtung nach einem der vorhergehenden Ansprüche,
**dadurch gekennzeichnet,**
**dass** eine Steuerung und/oder Regelung vorhanden ist;
wobei die Steuerung und/oder Regelung in der Lage ist, die Bedingungen im Kultivierungsbehälter (110) anhand von Daten der Sensoren (220) des Wärmeraums (100) zu beeinflussen;
wobei die Daten des Temperatursensors zur Steuerung und/oder Regelung der Heizelemente (210) des Wärmeraums (100) dienen;
wobei die Daten des CO₂-Sensors (220) zur Steuerung und/oder Regelung der mindestens einen Gaszufuhr dienen; und
wobei die Daten des Luftfeuchtigkeits-Sensors zur Ausgabe von Warnmeldungen dienen.

14. Abgedichteter Kultivierungsbehälter (110) zum Aufnehmen von Zellkulturen, mit mindestens einem abdichtbaren Durchlass (130; 310) für einen Sensor;
wobei der mindestens eine abdichtbare Durchlass (130; 310) in Kommunikation mit der Atmosphäre im Kultivierungsbehälter (110) steht;
mindestens einem abdichtbaren Gas-Ein- und/oder Auslass (330), der mit einem Gasanschluss (240) koppelbar ist; und
einer Position für ein Wasserreservoir (120) im Inneren des Kultivierungsbehälters (110),

15. Kultivierungsbehälter (110) nach dem unmittelbar vorhergehenden Anspruch zur Verwendung in einer Vorrichtung gemäß einem der Ansprüche 1 bis 13.

16. Verwendung eines Kultivierungsbehälters (110) nach Anspruch 14 in einer Vorrichtung gemäß einem der Ansprüche 1 bis 13.

## Claims

1. Device for providing a sterile environment for the incubation of cell cultures comprising:
a sealed warming chamber (100);
wherein the warming chamber (100) comprises at least one heating element (210);
wherein the warming chamber (100) comprises sensors for temperature and/or humidity and/or CO2 (220);
wherein the warming chamber (100) has at least one connection for a gas supply on the outside, wherein the connection is connected to a line that provides a gas connection (130; 240) in the interior of the warming chamber;
a sealed cultivation container (110) for holding the cell cultures;
wherein the cultivation container (110) is located in the warming chamber (100);
wherein the cultivation container (110) has a sealable passage (130; 310) in communication with the atmosphere in the cultivation container for each of said sensors of the warming chamber;
wherein the cultivation container (110) comprises at least one sealable gas inlet and/or outlet (130; 330) that is couplable to the at least one gas port (130; 240) in the interior of the warming chamber;
wherein the sensors of the warming chamber (100) are arranged to be introduced into the respective sealable passages (130; 310) of the warming chamber (100) upon insertion of the cultivation container (110) into the warming chamber (100); and
a water reservoir (120);
wherein the water reservoir (120) is arranged in the cultivation container (110).

2. Device according to the preceding claim,
**characterized in that**
each of said sensors of the warming chamber (100) is covered by a sterile filter.

3. Device according to any one of the preceding claims,
**characterized in that**
the at least one connection (240) of the warming chamber for a gas supply, the line connected thereto or the gas connection (130) connected via this line and provided in the interior of the warming chamber comprises a sterile filter.

4. Device according to any one of the preceding claims,
**characterized in that**
the warming chamber (100) has a tightly closing lid or door (250);
the opening of the lid or door (250) has a cross-section that is larger than a width of the cultivation container (110).

5. Device according to any one of the preceding claims,
**characterized in that**
each sealable passage (130; 310) on the cultivation container (110) comprises a sterile filter (320).

6. Device according to any one of the preceding claims,
**characterized in that**
the at least one sealable gas inlet and/or outlet (130; 330) on the cultivation container (110) comprises a sterile filter (340).

7. Device according to any one of the preceding claims,
**characterized in that**
the cultivation container (110) is a disposable element.

8. Device according to any one of the preceding claims,
**characterized in that**
the cultivation container (110) has a tightly closing lid or a tightly closing door (350).

9. Device according to any one of the preceding claims,
**characterized in that**
the water reservoir (120) is covered with a semi-permeable membrane (410).

10. Device according to the immediately preceding claim,
**characterized in that**
the semi-permeable membrane (410) of the water reservoir (120) is sealed prior to use with a film (420) impermeable to water and water vapor, which is removed for use.

11. Device according to any one of the preceding claims,
**characterized in that**
the water reservoir (120) contains sterile ultrapure water.

12. Device according to any one of the preceding claims,
**characterized in that**
the water reservoir (120) is a disposable element.

13. Device according to any one of the preceding claims,
**characterized in**
**that** a control and/or regulation system is provided;
wherein the control and/or regulation is capable of influencing the conditions in the cultivation container (110) based on data from the sensors (220) of the warming chamber (100);
wherein the temperature sensor data is used to control and/or regulate the heating elements (210) of the warming chamber (100);
wherein the data of the CO2 sensor (220) serves to control and/or regulate the at least one gas supply; and
wherein the data of the humidity sensor serve to output warning messages.

14. Sealed cultivation container (110) for holding cell cultures,
**comprising**
at least one sealable passage (130; 310) for a sensor;
wherein the at least one sealable passage (130; 310) is in communication with the atmosphere in the cultivation container (110);
at least one sealable gas inlet and/or outlet (330) couplable to a gas port (240); and
a location for a water reservoir (120) inside the cultivation container (110).

15. A cultivation container (110) according to the immediately preceding claim for use in a device according to any one of claims 1 to 13.

16. Use of a cultivation container (110) according to claim 14 in a device according to any one of claims 1 to 13.

## Revendications

1. Dispositif destiné à assurer un environnement stérile pour l'incubation de cultures cellulaires, comprenant :
un espace de chauffage étanche (100) ;
dans lequel l'espace de chauffage (100) comprend au moins un élément chauffant (210) ;
dans lequel l'espace de chauffage (100) comprend des capteurs de température et/ou d'humidité et/ou de CO2 (220) ;
dans lequel l'espace de chauffage (100) comprend à l'extérieur au moins un raccord pour une alimentation en gaz, le raccord étant relié à une conduite qui fournit un raccord de gaz (130 ; 240) à l'intérieur de l'espace de chauffage ;
un récipient de culture étanche (110) destiné à contenir les cultures cellulaires ;
dans lequel le récipient de culture (110) est disposé dans l'espace de chauffage (100) ;
dans lequel le récipient de culture (110) comprend, pour chacun desdits capteurs de l'espace de chauffage, un passage pouvant être rendu étanche (130 ; 310) qui est en communication avec l'atmosphère dans le récipient de culture ;
dans lequel le récipient de culture (110) comprend au moins un orifice d'entrée et/ou de sortie de gaz (130 ; 330) pouvant être rendu étanche et pouvant être couplé à l'au moins un orifice de gaz (130 ; 240) à l'intérieur de l'espace de chauffage ;
dans lequel les capteurs de l'espace de chauffage (100) sont disposés de manière à être insérés dans les passages respectifs (130 ; 310) pouvant être rendus étanches de l'espace de culture lorsque le récipient de culture (110) est inséré dans l'espace de chauffage (100) ; et
un réservoir d'eau (120) ;
dans lequel le réservoir d'eau (120) est disposé dans le récipient de culture (110).

2. Dispositif selon la revendication précédente,
**caractérisé en ce**
**que** chacun desdits capteurs de l'espace thermique (100) est recouvert par un filtre stérile.

3. Dispositif selon l'une quelconque des revendications précédentes,
**caractérisé en ce**
**que** l'au moins un raccord (240) de l'espace de chauffage pour une alimentation en gaz, la conduite qui y est reliée ou la prise de gaz (130) reliée par cette conduite et fournie à l'intérieur de l'espace de chauffage comprend un filtre stérile.

4. Dispositif selon l'une quelconque des revendications précédentes,
**caractérisé en ce**
**que** l'espace de chauffage (100) comprend un couvercle ou une porte (250) à fermeture étanche ;
dans lequel l'ouverture du couvercle ou de la porte (250) a une section transversale supérieure à une largeur de la cuve de culture (110).

5. Dispositif selon l'une quelconque des revendications précédentes,
**caractérisé en ce**
**que** chaque passage pouvant être fermé de manière étanche (130 ; 310) sur le récipient de culture (110) comprend un filtre stérile (320).

6. Dispositif selon l'une quelconque des revendications précédentes,
**caractérisé en ce**
**que** l'au moins une entrée et/ou sortie de gaz (130 ; 330) pouvant être rendue étanche sur le récipient de culture (110) comprend un filtre stérile (340).

7. Dispositif selon l'une quelconque des revendications précédentes,
**caractérisé en ce**
**que** le récipient de culture (110) est un élément jetable.

8. Dispositif selon l'une quelconque des revendications précédentes,
**caractérisé en ce**
**que** le récipient de culture (110) comprend un couvercle ou une porte (350) à fermeture étanche.

9. Dispositif selon l'une quelconque des revendications précédentes,
**caractérisé en ce**
**que** le réservoir d'eau (120) est recouvert d'une membrane semi-perméable (410).

10. Dispositif selon la revendication immédiatement précédente,
**caractérisé en ce**
**que** la membrane semi-perméable (410) du réservoir d'eau (120) est scellée, avant utilisation, par un film (420) imperméable à l'eau et à la vapeur d'eau, qui est retiré pour l'utilisation.

11. Dispositif selon l'une quelconque des revendications précédentes,
**caractérisé en ce**
**que** le réservoir d'eau (120) contient de l'eau pure stérile.

12. Dispositif selon l'une quelconque des revendications précédentes,
**caractérisé en ce**
**que** le réservoir d'eau (120) est un élément jetable.

13. Dispositif selon l'une des revendications précédentes,
**caractérisé en ce**
**qu'**il existe un système de commande et/ou de régulation ;
dans lequel la commande et/ou le contrôle est capable d'influencer les conditions dans le réservoir de culture (110) à partir des données des capteurs (220) de l'espace de chauffage (100) ;
dans lequel les données du capteur de température servent à la commande et/ou à la régulation des éléments chauffants (210) de l'espace de chauffage (100) ;
dans lequel les données ducapteur de CO2(220) servent à la commande et/ou à la régulation de l'au moins une alimentation en gaz ; et
dans lequel les données du capteur d'humidité servent à émettre des messages d'avertissement.

14. Récipient de culture étanche (110) pour contenir des cultures de cellules, comprenant au moins un passage pouvant être rendu étanche (130 ; 310) pour un capteur ;
dans lequel ledit au moins un passage pouvant être rendu étanche (130 ; 310) est en communication avec l'atmosphère dans le récipient de culture (110) ;
au moins un orifice d'entrée et/ou de sortie de gaz pouvant être rendu étanche (330) pouvant être couplé à un orifice de gaz (240) ; et
une position pour un réservoir d'eau (120) à l'intérieur du récipient de culture (110).

15. Récipient de culture (110) selon la revendication immédiatement précédente, destiné à être utilisé dans un dispositif selon l'une quelconque des revendications 1 à 13.

16. Utilisation d'un récipient de culture (110) selon la revendication 14 dans un dispositif selon l'une quelconque des revendications 1 à 13.
